# EUROPEAN PATENT SPECIFICATION

(11) **EP 2 723 853 B1**
(45) Date of publication and mention of the grant of the patent: **27.12.2017**
(21) Application number: 12803040.0
(22) Date of filing: 14.06.2012
(51) Int. Cl.: C12N 5/0775, C12N 5/077, A61K 35/28, A61K 35/34, G01N 33/50, A61P 9/00

(54) **IN VITRO CARDIOVASCULAR MODEL**
KARDIOVASKULÄRES IN-VITRO-MODELL
MODÈLE CARDIOVASCULAIRE IN VITRO

(30) Priority: 23.06.2011 FI 20115670
(43) Date of publication of application: 30.04.2014
(73) Proprietor: Tampereen yliopisto, 33014 Tampereen yliopisto (FI)
(72) Inventor: AALTO-SETÄLÄ, Katriina, FI-33014 Tampereen yliopisto (FI); HEINONEN, Tuula, FI-33014 Tampereen yliopisto (FI); KERKELÄ, Erja, FI-00310 Helsinki (FI); SARKANEN, Jertta-Riina, FI-33014 Tampereen yliopisto (FI); VUORENPÄÄ, Hanna, FI-33014 Tampereen yliopisto (FI); YLIKOMI, Timo, FI-33014 Tampereen yliopisto (FI)
(74) Representative: Kolster Oy Ab
(86) International application number: PCT/FI2012/050611
(87) International publication number: WO 2012/175797

(56) References cited:
- EP-A1- 2 184 068
- RU-C1- 2 334 793
- US-A1- 2007 116 674
- US-A1- 2010 022 005
- US-A1- 2010 035 297
- MERFELD-CLAUSS, S. ET AL.: 'Adipose tissue progenitor cells directly interact with endothelial cells to induce vascular network formation' TISSUE ENGINEERING: PART A vol. 16, no. 9, September 2010, pages 2953 - 2966, XP055140634
- CAO, Y. ET AL.: 'Human adipose tissue-derived stem cells differentiate into endothelial cells in vitro and improve postnatal neovascularization in vivo' BIOCHEMICAL AND BIOPHYSICAL RESEARCH COMMUNICATIONS vol. 332, no. 2, July 2005, pages 370 - 379, XP027230009
- KIM, Y.J. ET AL.: 'Role of CD9 in proliferation and proangiogenic action of human adipose-derived mesenchymal stem cells' PFLÜGERS ARCHIV EUROPEAN JOURNAL OF PHYSIOLOGY vol. 455, no. 2, November 2007, pages 283 - 296, XP019563530
- RUBINA, K. ET AL.: 'Adipose stromal cells stimulate angiogenesis via promoting progenitor cell differentiation, secretion of angiogenic factors, and enhancing vessel maturation' TISSUE ENGINEERING: PART A vol. 15, no. 8, August 2009, pages 2039 - 2050, XP055140638
- IKONEN, L. ET AL.: 'Vascularized heart tissue model for cardiac toxicity testing' CARDIOVASCULAR RESEARCH vol. 93, no. 1, March 2012, page S82, XP008172856
- HEYDARKHAN-HAGVALL SEPIDEH ET AL: "Human adipose stem cells: a potential cell source for cardiovascular tissue engineering", CELLS TISSUES ORGANS : IN VIVO, IN VITRO ; CTO, vol. 187, no. 4, 14 January 2008 (2008-01-14), pages 263-274, XP009182620, ISSN: 1422-6421, DOI: 10.1159/000113407 [retrieved on 2008-01-14]

## Description

### FIELD OF THE INVENTION

The present invention relates to an *in vitro* cardiovascular model for use in pharmacological studies. Furthermore, the invention relates to a method for the preparation of said model, and to a method of determining a biological activity of a test substance in said cardiovascular model. Still further, the invention relates to an implantable cardiac structure for use in the treatment of cardiac disorders.

### BACKGROUND OF THE INVENTION

A cardiovascular system together with respiratory and central nervous systems belongs to the vital organs or systems, the function of which is acutely critical for life. Therefore, chemical substances such as pharmaceuticals, industrial chemicals, biocides, food and feed preservatives and cosmetics have to be assessed for cardiac toxicity. These studies mainly involve the use of animals although animal-based tests have often been demonstrated to be poor models for predicting effects in man. Furthermore, animal tests are ethically questionable, costly and time consuming. For these reasons the strategy of both European Commission and US regulatory bodies is that the safety testing should be performed in non-animal models, and tests should be based on the predictive, toxicity pathway based human cell organotypic models that mimic as closely as possible the conditions in man (Toxicity Testing in the 21st Century: A Vision and a Strategy, 2007).

Anomalies in the cardiac action potential - whether due to a congenital mutation or injury - can lead to human pathologies, especially arrhythmias. The cardiac adverse drug reactions are utmost important because they are typically serious and can be fatal, as was seen for various drugs that were removed from the market in the 1980s and 1990s. These fatalities prompted regulatory attention and the development of the ICH Guidelines S7B and E14, released in 2005. These guidelines formalized the nonclinical and clinical assessments of all investigative drug's proarrhythmic liability. Proarrhythmias due to druginduced QT prolongation are the second most common cause for drug withdrawal and have caused increasing concern. The QT interval is affected by the heart rate. The most common models used today in safety pharmacological studies with new pharmaceuticals are animal models and ex vivo models that contains isolated hearts from guinea pig or rabbit or Purkinje cells isolated from a dog. No validated *in vitro* heart model exists that could be used for these purposes.

A few different in vitro 3D-cardiac tissue constructs have been developed with both contractile properties and action potentials (Zimmermann et al., Circulation Research, 2002, 90:22; Akiyama et al., Int. J. Mol. Sci., 2010, 11:2910). The disadvantages of the existing research models are that they are based on animal biology (rat cells) and that the models can be kept functional only for a short period of time (a few days). Thus short-term effects can only be assessed. Therefore, in order to mimic the heart function (beating frequency, beating strength, electrical activity, different channel activities) relevant to man human cell based functional tissue construct with relevant biomarkers and physico-chemical conditions control and maintenance would be needed to be developed.

US 2009/0169521 discloses an artificial 3D cardiac structure for use in the treatment of cardiac disorders. The structure is obtained by co-seeding cardiomyocytes, endothelial cells, and fibroblasts on or within an artificial scaffold. As exogenous scaffolds may interfere with cell-to-cell interactions and cell assembly in a multi-layered tissue construct (Norotte et al. Biomaterials, 2009, 30: 5910), the disclosed cardiac structure would not be optimal for use in pharmacological toxicology studies.

RU 2 334 793 C1 and Rubina et al. (Tissue Engineering: Part A. Vol. 15, No. 8, 2009, pages 2039-2050) disclose a co-culture model of adipose-derived stromal cells (ASCs) with cells isolated from early postnatal hearts (cardiomyocyte fraction, CMF). In the model, ASCs stimulated formation of capillary-like structures not only from pre-existing endothelial cells in the CMF, but also by promoting endothelial differentiation of cardiac progenitor cells. When ASCs were cultured in the absence of CMF, no formation of capillary-like structures was detected.

EP 2 184068 discloses cardiac adipose-tissue derived adult stem cells (cardiac ADSCs) which are more committed towards cardiac lineage than a population of subcutaneous fat-derived stem cells from the same individual. Cocultures of cardiac ADSCs with rat neonatal cardiomyocytes were used to demonstrate the cardiogenic potential of the human cardiac ADSCs.

In addition to toxicity studies of chemical and biological substances, human cell based organotypic heart models would be needed in investigation of novel medicines for cardiovascular diseases. In Western countries, cardiovascular diseases are the most common cause of deaths with heart failure being one of the most common diseases. For this reasons there is an urgent need to develop medicines and tissue engineering treatments to repair heart function. One approach is to use stem cell therapy to repair infracted area. The goal of the stem cell therapy is to differentiate patients own stem cells to functional cardiomyocytes and transplant the cells for reparation of the damaged area of the myocardium. However, before stem cell therapy can be applied to clinical use more research is needed to better understanding of stem cell differentiation, proliferation and behaviour. Tissue engineering treatments would greatly benefit from a human cell based functional heart model including vascular support.

Thus, there is an identified need in the art for validated cardiac *in vitro* models.

### BRIEF DESCRIPTION OF THE INVENTION

In one aspect, the present invention provides an *in vitro* cardiovascular structure comprising a tubule forming platform, which comprises at least partly formed tubules, and cardiomyocytes. The tubule forming platform comprises human adipose stem cells (hASCs), optionally, in the absence of any exogenous matrix components or added biomaterials. In some embodiments, the platform further comprises tubule forming endothelial cells, such as human umbilical vein endothelial cells, human microvascular endothelial cells, human adipose stem cell derived endothelial cells, human embryonic stem cell derived endothelial cells, induced pluripotent stem cell derived endothelial cells, transdifferentiation derived endothelial cells, endothelial progenitor cells, or endothelial cells obtained by genetic modification.

In another aspect, the present invention provides an *in vitro* cardiovascular structure for use in treating a cardiac disease.

In a still further aspect, the present invention provides a method of producing the *in vitro* cardiovascular structure described above. The method comprises the steps of a) providing hASCs, cardiomyocytes, and, optionally, tubule forming endothelial cells; b) culturing said hASCs, optionally, with said tubule forming endothelial cells; c) culturing said cardiomyocytes on top of the culture formed in step b); and d) administering VEGF and FGF-2 to the cell culture formed in step c).

Furthermore, one aspect of the present invention relates to a method of determining a biological activity of a test substance. The method comprises the steps of: a) providing an *in vitro* cardiovascular structure described above; b) administering said test substance to said structure; c) determining the effect of the test substance in said structure; and d) comparing the effect determined in step c) to a corresponding effect determined in the absence of said test substance. In some embodiments, the biological activity to be determined is selected from the group consisting of cellular toxicity, tubule formation modulating activity, electrical properties such as rate of cardiomyocyte contraction, mechanical properties such as force of cardiomyocyte contraction orand basic cell metabolism.

One further aspect of the present invention provides a method of treating a cardiac disease in a patient in need thereof, comprising implanting a cardiac structure described above into said patient. Non-liming examples of said cardiac disease may be selected from the group consisting of coronary heart disease and dilated cardiomyopathy.

Other aspects, specific embodiments, objects, details, and advantages of the invention are set forth in the dependent claims, following drawings, detailed description and examples.

### BRIEF DESCRIPTION OF THE DRAWINGS

In the following the invention will be described in greater detail by means of preferred embodiments with reference to the attached drawings, in which

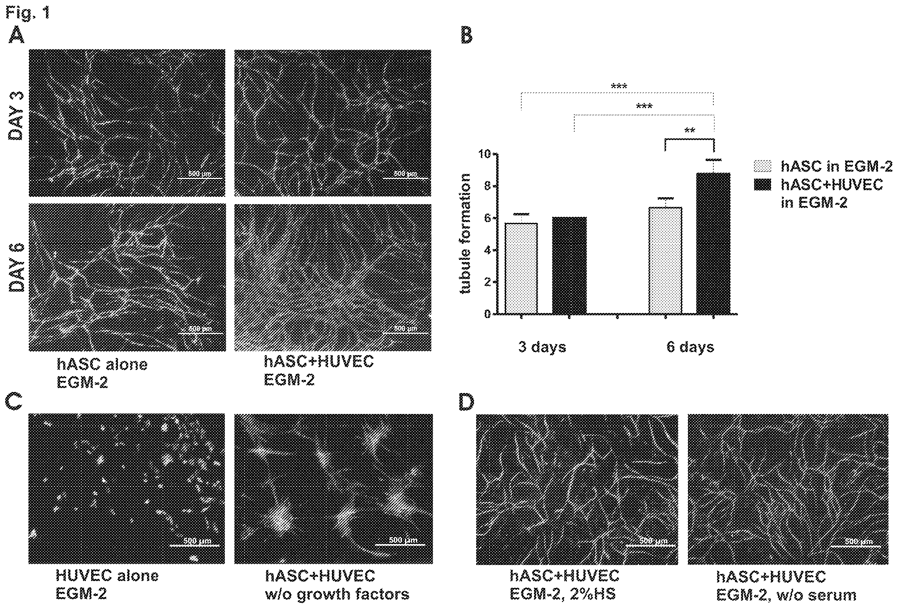

Figure 1 is a photograph illustrating the tubule formation of hASC monoculture and hASC+HUVEC co-culture. Cells were stained with von Willebrand factor antibody (anti-von Willebrand factor, 1:500, Sigma, red fluorescence shown with TRITC conjugated secondary antibody,1:100, Sigma). Figure 1A: Comparison of tubule formation of hASC monoculture and hASC+HUVEC co-culture. Cells were cultured and induced to angiogenesis for 3 or 6 days in growth factor enriched EGM-2 BulletKit medium. Figure 1B: Semi-quantitative analysis of the tubule formation between different treatments. hASC monoculture and hASC+HUVEC co-culture were compared to each other at 3 and 6 days. Semiquantitative scale according to Sarkanen et al., (Front. Pharmacol, 2011, 1: 147.). The results are reported as mean ± SD and differences considered significant when p<0.05*, p<0.01** and p<0.001***.
Figure 1C: For controls, HUVEC were plated at 4000 cells/cm² and grown in growth factor enriched EGM-2, and hASC+HUVEC co-culture grown without exogenous addition of growth factors Figure 1D: hASC+HUVEC were cultured in the growth factor enriched EGM-2 with human serum (EGM-2, 2% HS) or without serum (EGM-2 w/o serum).

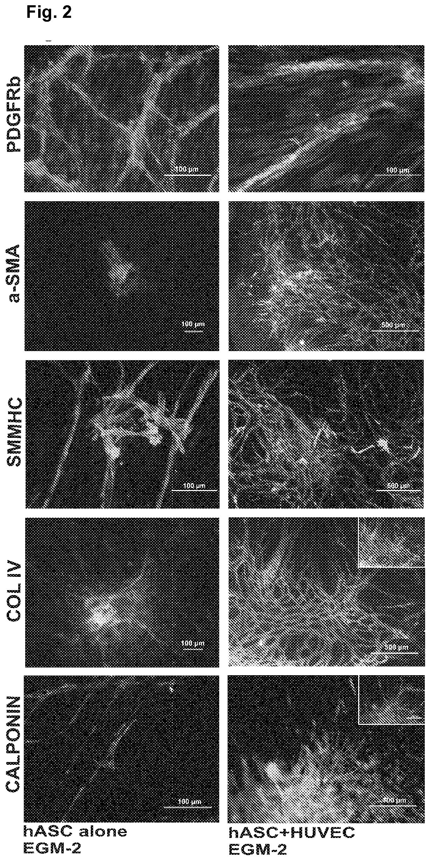

Figure 2 is a photograph illustrating the expression of pericytic and smooth muscle cell differentiation markers in tubule structures after angiogenic induction with growth factor enriched EGM-2 medium. For detection of tubule formation, cell cultures were immunostained with von Willebrand factor antibody (anti-von Willebrand factor, 1:500, Sigma, red fluorescence shown with TRITC conjugated secondary antibody, 1:100, Sigma). For detection of tubule maturation, cultures were immunostained with either anti- αSMA (1:200, Sigma), anti-COLIV (1:500, Sigma), anti-PDGFRβ (1:500, Sigma), anti-SMMHC (1:800, Sigma) or anti-calponin (1:800, Sigma), all of these green fluorescence, FITC-conjugated secondary antibody (1:100, Sigma). The images shown are merged images of double immunofluoresence at day 6, except for anti-PDGFRβ, that is at day 3, and except for anti-COLIV and anti-calponin for which both merged image of staining (small image) and the FITC-conjugated secondary antibody - anti-COLIV/anti-calponin staining (large images) are shown.

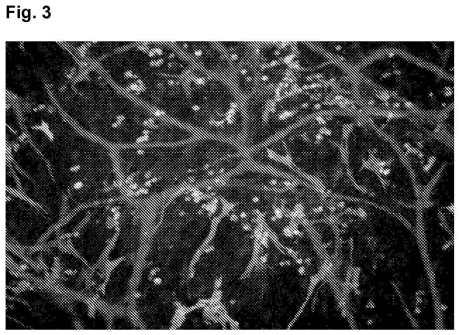

Figure 3 is a photograph illustrating an *In vitro* cardiovascular model based on hASCs, HUVECs and Neonatal Rat Cardiomyocytes, cultured for 10 days. Scale bar 100 µm. For detection of tubule formation, cell cultures were immunostained with von Willebrand factor antibody (anti-von Willebrand factor, 1:500, Sigma, TRITC conjugated secondary antibody, 1:100, Sigma). For detection of cardiomyocytes, the cultured were immunostained with cardiac specific anti-troponin T (1:500, Abcam) and FITC-conjugated secondary antibody (1:100, Sigma).

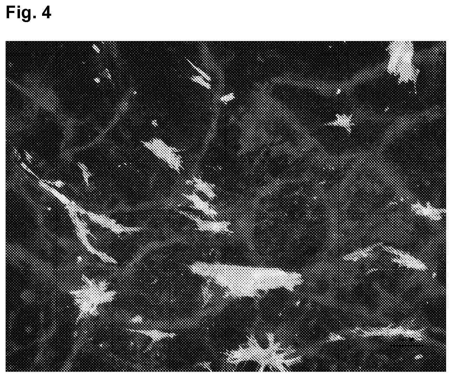

Figure 4 is a photograph illustrating an *In vitro* cardiovascular model based on hASCs, HUVECs and human embryonic stem cell derived cardiomyocytes, cultured for 10 days. Scale bar 100 µm. For detection of tubule formation, cell cultures were immunostained with von Willebrand factor antibody (anti-von Willebrand factor, 1:500, Sigma, TRITC conjugated secondary antibody, 1:100, Sigma). For detection of cardiomyocytes, the cultured were immunostained with cardiac specific anti-troponin T (1:500, Abcam) and FITC-conjugated secondary antibody (1:100, Sigma).

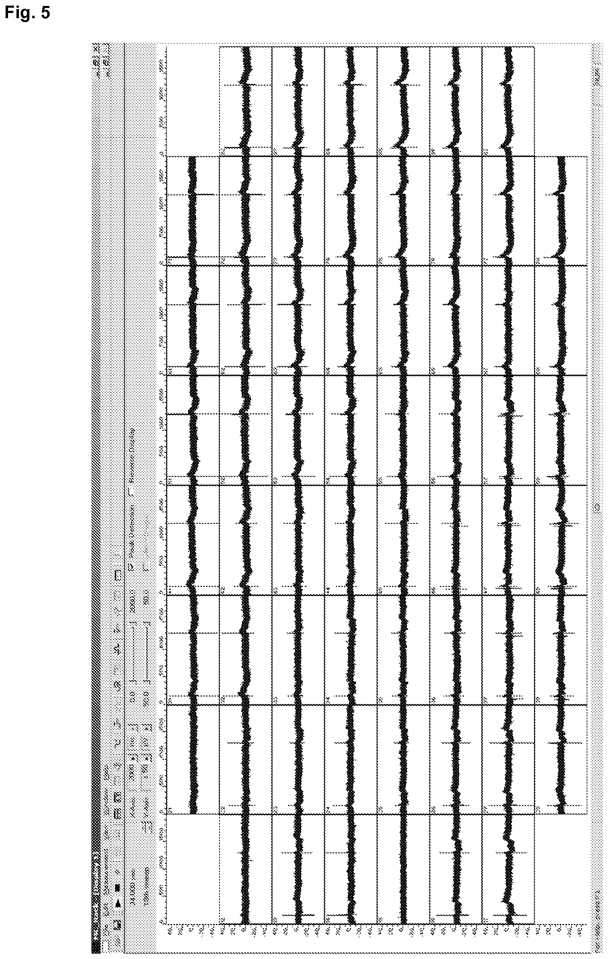

Figure 5 is a multi-electrode array (MEA) recording showing the electrical signal from synchronously contracting cardiovascular model 4 days after constructing the model.

### DETAILED DESCRIPTION OF THE INVENTION

The present invention provides an *in vitro* cardiovascular structure, i.e. a cardiovascular model, for use in pharmacological safety and toxicity studies. The model comprises cardiomyocytes cultured on a tubule forming platform comprising at least partly formed tubules. Surprisingly, such a functional cardiovascular model may be obtained without any exogenous matrix or added biomaterials.

Exogenous scaffolds typically used for creating multi-layered tissue constructs may interfere with cell-to-cell interactions and cell assembly. Thus, the present scaffold-free cardiovascular model is advantageous in this respect. An optimal tissue construct should contain no animal-derived components or unnatural scaffold materials and contain only growth factors and proteins that occur in tissues naturally. The present cardiovascular model, at least in some embodiments, fulfils these requirements and has features of mature vessels, i.e. in addition to the formation of tubule structures, the model is characterized by pericyte recruitment, basement membrane formation, and formation of a vessel supporting layer of smooth muscle cells.

As used herein, the terms "comprises" and "comprising" encompass the terms "consisting of" and "consisting essentially of".

As used herein, the term "tubule forming platform" refers to a multilayered cell structure having the capability of self-assembling into vascular network structures.

In one embodiment, the tubule forming platform may be constructed solely from adipose-derived stromal/stem cells (ASCs), such as human adipose stem cells (hASCs). This type of tubule forming platform is referred to as a monoculture model.

As used herein, the term "adipose-derived stem cell(s)" or "ASC(s)" refers to an unsorted stromal vascular fraction obtained from adipose tissue. Such a fraction is heterogeneous and comprises mesenchymal stem cells. According to more recent terminology, "ASCs" may also be referred to as "adipose-derived stromal cells". Methods of obtaining human ASCs (hASCs) are readily available in the art, including, but not limited to, the method disclosed in Example 1. ASCs have the ability to differentiate into a variety cell types, as well known in the art.

Herein is also disclosed a tubule forming platform constructed by co-culturing tubule forming endothelial cells and hASCs. This type of a tubule forming platform is referred to as a co-culture model.

As used herein, the term "tubule forming endothelial cell(s)" refers to endothelial cells having the capability of forming vascular structures, such as a vascular network. Non-limiting examples of tubule forming endothelial cells include human umbilical vein endothelial cells (HUVECs), human microvascular endothelial cells, human adipose stem cell derived endothelial cells, human embryonic stem cell derived endothelial cells, induced pluripotent stem cell derived endothelial cells, transdifferentiation derived endothelial cells, endothelial progenitor cells from other tissues, and endothelial cells obtained by genetic modification. Means and methods for inducing endothelial differentiation of the above-mentioned cells are readily known to a person skilled in the art. Tubule forming endothelial cells are also commercially available.

Embryonic stem cells (ESCs) are pluripotent cells having the ability to differentiate into a wide variety of different cell types, such as endothelial cells. WO 2007/130664 discloses a promising approach, termed blastomere biopsy, for obtaining human embryonic stem cells without damaging the donor embryo.

As used herein, the term "induced pluripotent stem cells" (iPSCs) refers to pluripotent stem cells generated from differentiated cells, typically from adult somatic cells such as fibroblasts by developmental reprogramming. Such cells have been described e.g. in WO 2008/151058 and US 2008/076176. Human induced pluripotent stem cells are referred to as hiPSCs.

As used herein, the term "transdifferentiation", or "lineage reprogramming", refers to a conversion of one mature cell type into another without undergoing an intermediate pluripotent state or progenitor cell type.

The tubule forming platform may be obtained by a method, wherein i) ASCs, or ii) ASCs and tubule forming endothelial cells, are plated on a cell culture or tissue culture plate, such as a 24-well, 48-well, 96-well plate or microplate in a cell culture medium supporting endothelial cell growth. A non-limiting example of such a culture medium is EGM-2 BulletKit™ obtainable from Lonza. The tubule formation may be induced in any endothelial growth medium (supplied by e.g. Lonza, Provitro, Promocell, BD); or in DMEM, DMEM/F12 or Knock-Out (KO) DMEM (supplied e.g. by Gibco Invitrogen, Sigma, BD, Lonza) that contain low concentration of human or animal serum, or no serum, and bFGF, VEGF, ascorbic acid, heparin, and/or hydrocortisone as supplements. Non-limiting examples of optional factors than may be further included are insulin, IGF-I, hEGF, transferrin and/or hormones such as trijodotyronine.

In some embodiments, the bottom surface of a cell culture plate may be grooved or scratched in order to align the tubules to be formed towards a desired orientation.

In the monoculture model, cells are typically but not necessarily plated in a density of about 24 x 10⁴ cells/cm² or more. In the co-culture model, ASCs and endothelial cells are typically but not necessarily plated in a ratio of 2:1 to 8:1, preferably 5:1, respectively. In one embodiment, ASCs are plated in a density of about 20 x 10⁴ cells/cm² and tubule forming endothelial cells in a density of about 4 x 10⁴ cells/cm². In some embodiments, the tubule forming cells, such as HUVECs, are plated 1 to 3 hours later that the ASCs, such as hASCs.

The present cardiovascular model is obtained by a method, wherein cardiomyocytes are seeded or plated on top of on a tubule forming platform thus modelling a human or animal heart. Non-limiting examples of cardiomyocytes suitable for use in the model include neonatal rat cardiomyocytes, human embryonic stem cell (hESC) derived cardiomyocytes, human induced pluripotent stem cell (hiPSC) derived cardiomyocytes, adult stem cell derived cardiomyocytes, human transdifferentiation-derived cardiomyocytes, and human cardiac myocytes. Means and methods for inducing cardiomyocyte differentiation are known in the art and include, but are not limited to, endodermal cell induced differentiation developed by Mummery et al. (Circulation, 2003, 107:2733), Activin A and BMP4 induced differentiation developed by Laflamme et al. (Nat Biotechnol, 2007, 25(9): 1015), and embryoid body technique developed by Kehat et al. (Circ. Res. 2002, 91: 659).

In some embodiments, cardiomyocytes are plated on top of a tubule forming platform in a complete serum-free medium (CSFM). This applies especially to rat cardiomyocytes. A non-limiting example of a commercially available cell culture medium suitable for use as a basal medium for CSFM is DMEM™ or DMEM/F12 or Knock-Out (KO) DMEM available from Gibco.

If the cardiovascular structure is implemented employing human cardiomyocytes, the culture medium may be serum-free or contain up to about 10% of bovine or human serum. For instance, DMEM/F12 may be used as a basal medium in such cases.

Typically, cardiomyocytes are plated in a density of about 1x10⁵ or about 2x10⁵ cells/cm², but the seeding density is not limited to these values.

Typically, but not necessarily, cardiomyocytes are seeded on top of a tubule forming platform one day later than the cells forming the tubule forming platform. It is not a prerequisite for the cardiovascular model that the tubules be completely formed prior to seeding the cardiomyocytes.

In order to follow real-time the tubule formation and cardiomyocyte alignment with the tubules, tubule forming endothelial cells and/or cardiomyocytes may be fluorescently labelled with e.g. lentivirus infection by inserting e.g. Green or Yellow Fluorescent Protein into the endothelial cell genome. The cells could also be genetically modified (including insertion of reporter genes, disease specific genes, differentiation related genes).

Typically, one day after plating the cardiomyocytes, vascular endothelial growth factor (VEGF) and basic fibroblast growth factor (FGF-2) are administered to the cells in order to induce tubule formation. This may be done by replacing the culture medium to fresh CSFM supplemented with said growth factors. Typically, VEGF is used in a concentration range of about 1 ng/ml to about 20 ng/ml, preferably 10 to 15 ng/ml; whereas FGF-2 is used typically in a concentration range of about 0,5 ng/ml to 2 ng/ml, preferably 1 to 2 ng/ml.

Optional agents for increasing angiogenic induction in the model include, but are not limited to, ascorbic acid, heparin, hydrocortisone, insulin-like growth factor 1 (IGF-1), epidermal growth factor (EGF), preferably human EGF, and any combinations thereof.

Further optional ingredients in a culture medium suitable for inducing angiogenesis (i.e. tubule formation) in the present model include bovine and human serum in a concentration up to 10%, as well as bovine and human albumin. As readily understood by a skilled person, any of the culture media used for producing and/or utilizing the disclosed tubule forming platform and/or the present cardiovascular model may contain any ingredients typically used in cell culture media, such as antibiotics, L-glutamine and sodium pyruvate.

Cardiomyocytes remain viable and functional in the cardiovascular model longer than previously possible. In some embodiments, contractility of the new born rat cardiomyocytes can be maintained three weeks compared to hiPSC and hESC derived cardiomyocytes which may be maintained for months. The viability and contractility of the cardiomyocytes is more important in the cardiovascular model than completely formed tubule structures. Thus, cardiovascular models with moderate tubule formation may be used for testing test substances according to various embodiments of the present invention.

In some embodiments, substances to be tested in the present cardiovascular model are added to the cells one day after administration of VEGF and FGF-2. A prerequisite is that the cardiomyocytes need to have functional properties before the chemical substances are added. The effects of said substances may be followed for e.g. two to three weeks, or even for months, if needed, depending on the application and source of cardiomyocytes.

Examples of biological effects to be determined include, but are not limited to, toxic effects as determined e.g. by assessing increase or decrease in the expression of different genes; viability of the cells by different means (e.g. MTT test, Neutral Red Uptake (NRU) assay, or LiveDead assay available from Invitrogen); electrical properties such as changes in the cardiomyocyte contraction rate and repolarization time or arrhythmic events as determined e.g. by measuring QT interval; mechanical properties such as changes in the contraction force as measured by different planar biosensors or distraction; immunostainings of cardiac markers such as connexin-43 for detecting GAP-junctions or markers such as cardiac specific troponin T; and changes in cell metabolism (e.g. lactic acid formation, calcium flux, changes in ion channels, glucose consumption, oxygen consumption, and carbon dioxide release). These effects may be assessed in any desired combination separately, sequentially, concomitantly, or simultaneously.

The cardiovascular model may contain one or more sensors, such as planar biosensors, for assessing any of the above-mentioned cellular effects. Suitable sensors include, but are not limited to, electrochemical, electrical and/or optical sensors. Further sensors may be included for monitoring and, if desired, adjusting physico-chemical properties of the culture medium.

As disclosed herein, the tubule forming platform *per se* may be used for assessing angiogenic properties of a test substance. Non-limiting examples of angiogenic properties to be assessed include tubule forming capability (e.g. by measuring tubule lengths and/or branches, or determining the presence of endothelial tight junctions) and tubule maturation capability (e.g. by determining the basement membrane formation, presence of pericytes and smooth muscle cells lining the mature tubule structures). In such cases, no cardiomyocytes are added to cell culture. The test substance may be applied to the tubule forming platform, for instance, one day after angiogenesis induction by VEGF and FGF-2 with or without above-mentioned optional angiogenesis inducing agents. The angiogenic properties may be followed for e.g. few days or two weeks. The test substance may be applied to the model even prior to the tubules being completely formed.

Non-limiting examples of test substances to be screened in the present cardiovascular and angiogenesis models include chemical and biological substances such as small molecule chemical compounds, nanoparticles, polypeptides, antibodies, and growth factors.

Although the cardiovascular structure and the tubule forming platform in the absence of any exogenous matrix components and added biomaterials functions well for the purposes of pharmacological safety and toxicity tests and mimics a cardiac tissue without interfering non-native components, it may, however, in some cases be advantageous to include such components in the model and/or the platform. Such embodiments may be used, for instance, to test safety and toxicity of test substances in an artificial cardiac construct. Non-limiting examples of suitable exogenous matrix components or biomaterials to be provided in the cardiovascular model and/or the tubule forming platform include, but are not restricted to, synthetic or natural polymers such as collagen I or IV, hyaluronic acid, gelatin or other extracellular matrix components.

In some aspects of the present invention, the cardiovascular structure which contains exogenous matrix components and/or added biomaterials may be constructed as an implantable 3D cardiac structure for use in the treatment of cardiac diseases including, but not limited to coronary heart disease and dilated cardiomyopathy.

As used herein, the term "treatment" or "treating" refers not only to complete cure of a disease, but also to prevention, alleviation, and amelioration of a disease or symptoms related thereto.

For therapeutic purposes, it is important that the cardiac structure is xeno-free, i.e. it does not contain any components obtained from a foreign source or is not prepared under conditions containing foreign agents. Furthermore, it may be advantageous to use of autologous cells for therapeutic purposes.

It will be obvious to a person skilled in the art that, as the technology advances, the inventive concept can be implemented in various ways. The invention and its embodiments are not limited to the examples described above but may vary within the scope of the claims.

### EXAMPLES

All work has been performed according to the guidelines of national Ethical Committee. The human umbilical cords were obtained from scheduled caesarean sections while human adipose tissue specimens were obtained from surgical operations, both with appropriate permissions and informed consents from Tampere University Hospital. Further, Pirkanmaa Hospital District ethical committee has approved the derivation and use of human iPS and hESC cells, permission Nos. R08070 and R051116, respectively.

### EXAMPLE 1. Construction and characterization of co-culture based tubule forming platform

### Isolation of human umbilical vein endothelial cells (HUVECs):

HUVECs cells were derived from umbilical cords obtained from scheduled cesarean sections with informed consent from Tampere University Hospital (permission No. R08028 from the Ethics Committee of the Pirkanmaa Hospital District, Tampere, Finland). The isolation of umbilical vein endothelial cells (HUVEC) from human umbilical cord veins was performed as described by Jaffe et al. (J Clin Invest, 1973, 52: 2745) but the process was further optimized. The umbilical cord was separated from the placenta and the umbilical vein was cannulated with a 20G needle. The needle was secured by clamping the cord over the needle with a surgical clamp. The vein was perfused with Umbilical cord buffer solution (UBS; 0.1 M phosphate buffer solution containing 0.14 M NaCl, 0.004 M KCI, and 0.011 M glucose) to wash out blood after which the other end of the umbilical vein was clamped with a surgical clamp. The vein was infused with 0.05% collagenase I. The umbilical cord was incubated in a water bath at 37°C for up to 20 min. After incubation, the collagenase I solution containing HUVEC was flushed from the cord by perfusion with PBS into a 50 ml polypropylene tube (Sarstedt). The cells were centrifuged at 200 x g for 10 min, washed once with medium, centrifuged again and resuspended in EGM-2 BulletKit medium (Lonza Group Ltd, Basel, Switzerland) and seeded into 75 cm² flasks. The cells were cultured at 37°C in 5% CO₂ incubator. Medium was changed every two to three days and cells were divided when confluent. For assay controls, HUVEC were plated at 4000 cells/cm² and cultured in EGM-2 BulletKit medium.

The isolated HUVEC were daily observed microscopically for their morphology, cell culture purity, and cell proliferation. The medium was changed every 2-3 days. When confluent, the cells were detached with Tryple Express. Pure HUVEC cultures with good proliferation capacity were subcultured at primary culture (p0) in the ratio of 1:2-1:4 and at passages 1 (p1) upward in a ratio of 1:3-1:5.

### Lentivirus infection:

Lentiviral construct pLKO-MISSION-Bright-GFP was purchased from Biomedicum Genomics (BMGen, Biomedicum Helsinki, Helsinki, Finland). The infection was carried out with HUVEC at low passages with 300 µl of pLKO-MISSION-Bright-GFP in 1 ml EGM-2 Bullet Kit medium (1 U/ml). Virus infection was accelerated with 8 µg/ml hexadimethrine bromide (Sigma). After 24 hours of incubation, medium was replaced with fresh EGM-2 medium. Highly fluorescent clones were selected with cloning rings and further selected with dilution cloning to obtain pure GFP-HUVEC-culture. After expanding the infected HUVEC, they were used for hASC and HUVEC co-culture assay as described below.

### Isolation of human adipose stem cells (hASCs):

Stem cell isolation procedure was performed as described previously (Gimble and Guilak, Cytotherapy, 2003, 5: 362; Hong et al. Mol Cell Biochem, 2005, 276.Niemela et al., J Craniofac Surg, 2007, 18: 325-335). Briefly, human adipose tissue specimens were cut into small pieces, enzymatically digested with 0,05% collagenase I (Invitrogen, Paisley, Scotland, UK) in Dulbecco's Modified Eagle's Medium Nutrient Mixture F-12 (DMEM/F12, Gibco, Invitrogen, Carlsbad, CA, USA) for 60 min at 37°C in a gyratory water bath. The digested tissue was centrifuged at 600 x g for 10 min in room temperature (RT). The digested tissue was filtered through a 100 µm filter (Sarstedt, Numbrecht, Germany), centrifuged and filtered through a 40 µm filter (Sarstedt). Cells were seeded into 75 cm² flasks (Nunc EasyFlask™, Nunc, Roskilde, Denmark) in DMEM/F12 supplemented with 1% L-glutamine (L-glut, Gibco), 1% Antibiotic-antimycotic mixture (AB/AM, Gibco) and 15% human serum (HS, Cambrex, East Rutherford, NJ, USA). The next day, cells were washed several times with PBS. The cells were maintained at 37°C under a 5% CO₂ air atmosphere at a constant humidity and medium was changed every two to three days. After grown to confluency, cells were divided in a ratio of 1:2-1:3, or further used for cell culture studies.

### Co-culture of hASCs and HUVECs:

Human ASC (up to passage 7) were seeded in EGM-2 BulletKit (Lonza) culture medium into 48-well plates (Nunclon™ Multidishes, Nunc, Roskilde, Denmark) at a density of 20 000 cells/cm². HUVEC, cultured as above (up to passage 4), were immediately carefully seeded on top of hASC at a density of 4000 cells/cm². The day after plating, VEGF (10 ng/ml) and FGF-2 (1 ng/ml) were applied to the co-culture.

Cells were cultured for either 3 or 6 days prior to immunocytochemistry or quantitative RT-PCR. Medium was changed and the treatments applied once to cells cultured for 3 days and twice to cells cultured for 6 days.

### Immunocytochemistry:

The tubule formation was visualized with endothelial cell specific antibody to von Willebrand Factor (anti-vWf primary antibody produced in rabbit, 1:500, Sigma). To evaluate human adipose stem cell differentiation, parallel double immunofluorescence staining with α-vWf was performed. Primary antibody against either common pericytic marker α-smooth muscle actin (monoclonal anti αSMA clone 1A4, 1:200, Sigma), vascular smooth muscle cell marker smooth muscle myosin heavy chain (anti-SMMHC, 1:800, Sigma), contractile smooth muscle cell marker calponin (anti-calponin, 1:800, Sigma), pericytic and smooth muscle cell progenitor marker platelet derived growth factor receptor-β (anti-PDGFRβ 1:800) or basement membrane marker collagen IV (anti-COLIV, 1:500, Sigma) was combined with anti-vWf. Cells were washed three times with PBS, fixed with ice-cold 70% ethanol for 20 minutes, permeabilized with 0,5% Triton X-100 (JT Baker, Phillipsburg, NJ, USA) for 15 minutes and blocked for unspecific staining with 10% bovine serum albumin (BSA, Sigma) for 30 minutes. After blocking, cells were incubated with the primary antibody pairs at 1 hour at RT. Cells were washed three times with PBS, incubated 30 min with secondary antibody polyclonal anti- rabbit IgG TRITC (1:100, Sigma) for anti-vWf and polyclonal anti- mouse IgG FITC (1:100, Sigma) for anti-aSMA, anti-COLIV, anti- PDGFR-β and anti-SMMHC. Cell nuclei were stained with Hoechst 33258 (1ug/ml, Sigma) for 5 minutes and washed 5 times with PBS. For anti-GFP staining, primary antibody pair was mouse monoclonal antibody to GFP (Abcam, Cambridge, UK, 1:100) and anti-vWf, secondary antibodies being anti- mouse IgG TRITC (Sigma, 1:100) and polyclonal antibody to rabbit IgG FITC, (Acris Antibodies GmbH, Hiddenhausen, Germany, 1:500), respectively. Fluorescence was visualized with Nikon Eclipse Ti-S microscope (Nikon, Tokyo, Japan) and the images were processed with Adobe Photoshop software 7.0 (Adobe Systems, San Jose, CA, USA) and Corel Draw software 10.0 (Corel Corporation, Ottawa, ON, Canada).

### Microscopic analysis of tubule formation:

After immunocytochemical staining, the tubules were analyzed with Nikon Eclipse TS100 microscope (Nikon, Tokyo, Japan) from 48-well plate wells with 40x magnification. The extent of tubules in different cultures was quantified visually by using semi-quantitative grading scale from 0 to 10, the grading was based on tubule formation, the length and the branches of tubules, as described in our previous study (Sarkanen et al., 2011).

### Statistical analysis:

Statistical analyses were performed and graphs processed with GraphPadPrism 5.0 (GraphPad Software, Inc., San Diego, CA, USA). Tubule formation and RT-PCR results were subjected to One-way ANOVA followed by Dunnett's and Bonferroni's post tests when applicable. The results were reported as mean ± SD and differences were considered significant when p<0.05*, p<0.01** and p<0.001***.

### Results:

The tubule formation capacity and anti-vWf-positive endothelial tubule structures of the co-culture were evaluated and compared at two different time points (day 3 and day 6). The co-culture showed early (day 3) tubular network formation which was reproducible and not dependent on the cell line or passage number of the cells. At day 6, the co-culture showed an extremely accelerated proliferation rate as massive, dense multilayered vascular network formation.

Semi-quantitative evaluation of the tubule formation was showed that the co-culture had significantly more tubules at day 6 than at day 3 (p<0.001). Control cells grown without growth factors, as well as HUVECs alone, grown in growth factor enriched EGM-2 medium, showed only mild tubule formation or no tubule formation, respectively.

The co-culture was also subjected to immunocytochemical staining. PDGFRβ expression was most intense at day 3 and was seen as dot-like structures surrounding the developing tubules constantly. At day 6, PDGFRβ was seen in some extent. COLIV, showing the development of basement membrane, was remarkably widely expressed in the co-culture. The expression was co-localized with the developing tubules, covering the tubules. α-SMA and SMMHC positive cells were expressed widely in the co-culture at day 6, often localized in the branching points of tubular structures and in between the tubules. SMMHC expression was increased between days 3 and 6. It can be concluded, that co-culture model forms a dense multilayered vascular network with properties of mature blood vessels such as complete basement membrane formation and smooth muscle cells with contractile properties aligning the tubules. This co-culture model is more mature that any of the previous developed angiogenesis models.

### EXAMPLE 2. Construction and characterization of monoculture based tubule forming platform

Human ASCs were obtained as described in Example 1 and seeded in EGM-2 BulletKit medium into 48-well plates (Nunclon™ Multidishes, Nunc, Roskilde, Denmark) at a density of 20 000 cells/cm². Cells were cultured for either 3 or 6 days in EGM-2 BulletKit medium, a commercially available growth factor enriched medium containing EGF, VEGF, bFGF, IGF-I, ascorbic acid, heparin, 0,1 % gentamicin/amphotericin-B and 2 % FBS, or in DMEM/F-12 medium supplemented with 15% HS, 1mM L-glut and 1% AB/AM. Medium was changed and the treatments applied once to cells cultured for 3 days and twice to cells cultured for 6 days. As assay control, hASC were cultured in DMEM/F-12 medium supplemented with 15% HS, 1mM L-glut and 1% AB/AM.

### Results:

In the hASC monoculture, the induction towards angiogenesis was not as massive as in the co-culture. However, in the monoculture, vessel supporting pericytic and smooth muscle cell markers were often seen.

### EXAMPLE 3. Construction and characterization of in vitro cardiovascular model

HUVECs and hASCs used in this Example were obtained as described in Example 1. Neonatal rat cardiomyocytes were extracted from neonatal rat puppies aged two to three days.

An *in vitro* cardiovascular model was constructed in a 48-well plate as follows:

### Day 0: construction of a tubule forming platform

Co-culture model: hASCs (up to passage 4) were seeded in EGM-2 BulletKit -medium into 48-well plates at a density of 20 000 cells/cm². After 1-3 hours, HUVECs (up to passage 4) in EGM-2 culture medium were carefully seeded on top of hASC at a density of 4000 cells/cm².

Monoculture model: hASCs (up to passage 4) were seeded in EGM-2 BulletKit -medium into 48-well plates at a density of 24 000 cells/cm².

### Day 1: construction of an in vitro cardiovascular model

Neonatal rat cardiomyocytes (100 000, 200 000, or 40000 cells) in complete serum free medium (CSFM) were seeded on top of the tubule forming platform.

### Day 2: Induction of differentiation

The medium was changed to CSFM supplemented with 10 ng/ml vascular endothelial growth factor (VEGF) and 1 ng/ml basic fibroblast growth factor (FGF-2). The medium was changed to a fresh one three times in a week.

### Results:

Neonatal rat cardiomyocytes survived viable and contractile in the monoculture model for about 7 days and for at least 14 days in the co-culture model (see Table 1). The striated form of the cell morphology was maintained throughout the culture time. The cardiomyocytes were orientated along or close to the tubule structures and were synchronously contracting throughout the culture.

**Table 1. Contractility of neonatal rat cardiomyocytes**

| **Cell number** | **Follow-up time and level of cardiomyocyte contractility cultured alone*** | **Follow-up time and level of cardiomyocyte contractility in tubule forming co-culture*** |
|---|---|---|
| 0,1 x 10⁶ | 7 days, moderate | 14 days, strong |
| 0,2 x 10⁶ | 7 days, moderate | 14 days, strong |
| 0,4 x 10⁶ | 12 days, strong | 14 days, strong |

| | | |
|---|---|---|
| * approximations done by visual inspection | | |

(EGM-2 BulletKit Single Quots supplements, Lonza) and heparin (EGM-2 Single Quots supplements, Lonza).

Medium 3: CSFM supplemented with 2 % FBS (fetal bovine serum. Gibco), 10 ng/ml VEGF and 1 ng/ml FGF-2.

Medium 4: Angiogenic stimulation medium: Endothelial cell basal medium (EBM-2, Lonza) supplemented with 10 ng/ml VEGF, 1 ng/ml FGF-2, 0,1 % gentamicin (GA-1000, Lonza), 2 % fetal bovine serum and 1 mM L-glutamine.

Medium 5: Angiogenic stimulation medium + human serum: Endothelial cell basal medium (EBM-2, Lonza) supplemented with 10 ng/ml vascular endothelial growth factor,and 1 ng/ml basic fibroblast growth factor (FGF-2, Sigma), 0,1 % gentamicin (GA-1000, Lonza) and 2 % human serum (Lonza) serum and 1 mM L-glutamine.

### EXAMPLE 4. Comparative results

Tubule formation and cardiomyocyte contractibility of neonatal rat cardiomyocytes (NRC) was assessed in a co-culture based tubule forming platform seven different treatments.

Medium 1: CSFM (complete serum free medium) supplemented with 10 ng/ml VEGF (Sigma Aldrich, Manassas, VA, USA) and 1 ng/ml FGF-2 (Sigma).

50 ml of CSFM was composed of the following ingredients:
- DMEM/F-12 42 ml
- 200 mM L-glutamine 0.64 ml
- 100 x penicillin/streptomycin 0,5 ml
- 0.1 nM T3 0.5 µl
- 10 x BSA 5 ml
- 100 mM Sodium pyruvate 1. 4 ml
- ITS 0.576 ml

Medium 2: CSFM supplemented with 10 ng/ml VEGF, 1 ng/ml FGF-2, ascorbic acid (EGM-2 Single Quots supplements, Lonza), hydrocortison Medium 6: EGM-2 BulletKit -medium (Lonza) where 2 % fetal bovine serum (Lonza) is replaced by 2 % human serum (Lonza).

Medium 7: EGM-2 BulletKit -medium (Lonza) without fetal bovine serum.

**Table 2. Cardiomyocyte contractibility and tubule formation**

| **Treatment** | **NRC + Angiogenesismodel (HUVEC+hASC cells)** | |
|---|---|---|
| | Survival time and level of cardiomyocyte contractility* | Tubule formation ** |
| Medium 1: | 14 days, strong | 2 |
| CSFM +VEGF+ FGF | | |
| Medium 2: | 13 days, strong | 7 |
| CSFM+ VEGF+ FGF | | |
| +ascorbic acid | | |
| + hydrocortisone | | |
| + heparin | | |
| Medium 3: | 10 days, moderate | 2 |
| CSFM + VEGF + FGF | | |
| + 2 % FBS | | |
| Medium 4: | 2 days, weak | 2 |
| Angiogenic stimulation | | |
| medium | | |
| Medium 5: | 6 days, weak | 3 |
| Angiogenic stimulation media | | |
| + human serum | | |
| Medium 6: | 4 days, weak | 8 |
| EGM-2 | | |
| + human serum | | |
| Medium 7: | 3 days, weak | 8 |
| EGM-2 without serum | | |

| | | |
|---|---|---|
| * by visual inspection **at scale 1-8 according to Sarkanen et al. 2011 | | |

### EXAMPLE 5. Construction and characterization of in vitro human cardiovascular model

HUVECs and hASCs used in this Example were obtained as described in Example 1. Human embryonic stem cell derived cardiomyocytes were differentiated for 2 weeks as described by Mummery et al.(*ibid*).. The beating clusters were cut out, dissociated and cultured in DMEM/F12 supplemented with 10% FBS, 1% NEAA and 1% Glutamax (EB medium).

An *in vitro* cardiovascular model was constructed in a 48-well plate as follows:

### Day 0: construction of a tubule forming platform

Co-culture model: hASCs (up to passage 4) were seeded in EGM-2 BulletKit -medium into 48-well plates at a density of 20 000 cells/cm². After 1-3 hours, HUVECs (up to passage 4) in EGM-2 culture medium were carefully seeded on top of hASC at a density of 4000 cells/cm².

Monoculture model: hASCs (up to passage 4) were seeded in EGM-2 BulletKit -medium into 48-well plates at a density of 24 000 cells/cm².

### Day 1: construction of an in vitro cardiovascular model

Human embryonic stem cell derived cardiomyocytes (1-7 cell aggregates per 48-well plate well) in their EB were seeded on top of the tubule forming platform.

### Day 2: Induction of differentiation

The medium was changed to EB supplemented with 10 ng/ml vascular endothelial growth factor (VEGF) and 1 ng/ml basic fibroblast growth factor (FGF-2). The medium was changed to a fresh one three times in a week.

### Results:

Figure 4 illustrates that human cardiomyocytes were functional i.e. contractile and presented typical morphology of mature-like cardiomyocytes even after 10 days in the co-culture model.

## Claims

1. An *in vitro* cardiovascular structure comprising
cardiomyocytes seeded on top of a tubule forming platform which is constructed from human adipose stem cells (hASCs) and comprises at least partly formed tubules prior to seeding said cardiomyocytes.

2. The structure according to claim 1, wherein the tubule forming platform further comprises endothelial cells.

3. The structure according to claim 2, wherein the endothelial cells are selected from the group consisting of human umbilical vein endothelial cells, human microvascular endothelial cells, human adipose stem cell derived endothelial cells, human embryonic stem cell derived endothelial cells, induced pluripotent stem cell derived endothelial cells, transdifferentiation derived endothelial cells, endothelial progenitor cells, and endothelial cells obtained by genetic modification.

4. The structure according to any one of claims 1 to 3, wherein said cardiomyocytes are selected from the group consisting of new born rat cardiomyocytes, hiPSC derived cardiomyocytes, hESC derived cardiomyocytes, adult stem cell derived cardiomyocytes, transdifferentation-derived cardiomyocytes, and human primary cardiomyocytes.

5. The structure according to any one of claims 1 to 4, further comprising exogenous matrix components or added biomaterials selected from the group consisting of synthetic polymers, natural polymers, collagen I, collagen IV, hyaluronic acid, gelatin and other extracellular matrix components, or mixtures thereof.

6. The structure according to any one of claims 1 to 5 for use in treating a cardiac disease, preferably selected from the group consisting of coronary heart disease and dilated cardiomyopathy.

7. A method of producing an *in vitro* cardiovascular structure according to claim 1, comprising:
a) providing hASCs, cardiomyocytes, and, optionally, endothelial cells;
b) culturing said hASCs, optionally, with said endothelial cells;
c) culturing said cardiomyocytes on top of the culture formed in step b); and
d) administering VEGF and FGF-2 to the cell culture formed in step c).

8. The method according to claim 7, wherein the endothelial cells are selected from the group consisting of human umbilical vein endothelial cells, human microvascular endothelial cells, human adipose stem cell derived endothelial cells, human embryonic stem cell derived endothelial cells, induced pluripotent stem cell derived endothelial cells, transdifferentiation derived endothelial cells, and endothelial progenitor cells.

9. A method of determining a biological activity of a test substance, comprising the steps of:
a) providing an *in vitro* cardiovascular structure according to claim 1;
b) administering said test substance to said structure or platform;
c) determining the effect of the test substance in said structure or platform; and
d) comparing the effect determined in step c) to a corresponding effect determined in the absence of said test substance.

10. The method according to claim 9 wherein the biological activity to be determined is selected from the group consisting of cellular toxicity, tubule formation modulating activity, electrical properties such as rate regularity of cardiomyocyte contraction, duration of repolarization time, presence of arrhythmogenicity, mechanical properties such as force of cardiomyocyte contraction and cell metabolism.

## Patentansprüche

1. Kardiovaskuläre *in vitro*-Struktur, umfassend
Kardiomyozyten, beimpft auf das Obere von einer Tubulus bildenden Plattform, die aus humanen Fettstammzellen (hASCs) aufgebaut ist und mindestens teilweise gebildete Tubuli vor dem Beimpfen der Kardiomyozyten umfasst.

2. Struktur nach Anspruch 1, wobei die Tubulus bildende Plattform weiter endotheliale Zellen umfasst.

3. Struktur nach Anspruch 2, wobei die endothelialen Zellen ausgewählt sind aus der Gruppe, bestehend aus humanen endothelialen Nabelvenen-Zellen, humanen endothelialen Mikrogefäß-Zellen, von humaner Fettstammzelle abgeleiteten endothelialen Zellen, von humaner embryonaler Stammzelle abgeleiteten endothelialen Zellen, von induzierter pluripotener Stammzelle abgeleiteten endothelialen Zellen, von Transdifferenzierung abgeleiteten endothelialen Zellen, endothelialen Progenitorzellen und endothelialen Zellen, erhalten durch genetische Modifizierung.

4. Struktur nach einem der Ansprüche 1 bis 3, wobei die Kardiomyozyten ausgewählt sind aus der Gruppe, bestehend aus Kardiomyozyten von neugeborener Ratte, von hiPSC abgeleiteten Kardiomyozyten, von hESC abgeleiteten Kardiomyozyten, von adulter Stammzelle abgeleiteten Kardiomyozyten, Transdifferentation-abgeleiteten Kardiomyozyten und humanen primären Kardiomyozyten.

5. Struktur nach einem der Ansprüche 1 bis 4, weiter umfassend exogene Matrix-Komponenten oder zugegebene Biomaterialien, ausgewählt aus der Gruppe, bestehend aus synthetischen Polymeren, natürlichen Polymeren, Kollagen I, Kollagen IV, Hyaluronsäure, Gelatine und anderen extrazellulären Matrix-Komponenten oder Gemischen davon.

6. Struktur nach einem der Ansprüche 1 bis 5 zur Verwendung beim Behandeln einer Herzkrankheit, vorzugsweise ausgewählt aus der Gruppe, bestehend aus koronarer Herzerkrankung und dilatierter Kardiomyopathie.

7. Verfahren zur Herstellung einer kardiovaskulären *in vitro*-Struktur nach Anspruch 1, umfassend:
a) Bereitstellen von hASCs, Kardiomyozyten und gegebenenfalls endothelialen Zellen;
b) Züchten der hASCs, gegebenenfalls mit den endothelialen Zellen;
c) Züchten der Kardiomyozyten auf dem Oberen der in Schritt b) gebildeten Kultur; und
d) Verabreichen von VEGF und FGF-2 an die in Schritt c) gebildete Zellkultur.

8. Verfahren nach Anspruch 7, wobei die endothelialen Zellen ausgewählt sind aus der Gruppe, bestehend aus humanen endothelialen Nabelvenen-Zellen, humanen endothelialen Mikrogefäß-Zellen, von humaner Fettstammzelle abgeleiteten endothelialen Zellen, von humaner embryonaler Stammzelle abgeleiteten endothelialen Zellen, von induzierter pluripotener Stammzelle abgeleiteten endothelialen Zellen, von Transdifferenzierung abgeleiteten endothelialen Zellen und endothelialen Progenitorzellen.

9. Verfahren zum Bestimmen einer biologischen Wirkung von einer Testsubstanz, umfassend die Schritte:
a) Bereitstellen einer kardiovaskulären *in vitro*-Struktur nach Anspruch 1;
b) Verabreichen der Testsubstanz an die Struktur oder Plattform;
c) Bestimmen des Effekts der Testsubstanz in der Struktur oder Plattform; und
d) Vergleichen des in Schritt c) bestimmten Effekts mit einem entsprechenden in Abwesenheit der Testsubstanz bestimmten Effekt.

10. Verfahren nach Anspruch 9, wobei die zu bestimmende biologische Aktivität ausgewählt ist aus der Gruppe, bestehend aus zellulärer Toxizität, Tubulus-Bildungsmodulierender Aktivität, elektrischen Eigenschaften, wie Ratenregularität der Kardiomyozytenkontraktion, Dauer der Repolarisationszeit, Vorliegen von Arrhythmogenizität, mechanischen Eigenschaften, wie Kraft der Kardiomyozytenkontraktion und Zellmetabolismus.

## Revendications

1. Structure cardiovasculaire *in vitro* comprenant
des cardiomyocytes ensemencés au sommet d'une plateforme de formation de tubules qui est construite à partir de cellules souches adipeuses humaines (hASCs) et comprend des tubules au moins partiellement formés avant d'ensemencer lesdits cardiomyocytes.

2. Structure selon la revendication 1, dans laquelle la plateforme de formation de tubules comprend en outre des cellules endothéliales.

3. Structure selon la revendication 2, dans laquelle les cellules endothéliales sont sélectionnées dans le groupe constitué de cellules endothéliales de veine ombilicale humaine, de cellules endothéliales micro-vasculaires humaines, de cellules endothéliales dérivées de cellules souches adipeuses humaines, de cellules endothéliales dérivées de cellules souches embryonnaires humaines, de cellules endothéliales dérivées de cellules souches pluripotentes induites, de cellules endothéliales dérivées de transdifférenciation, de cellules progénitrices endothéliales, et de cellules endothéliales obtenues par modification génétique.

4. Structure selon l'une quelconque des revendications 1 à 3, dans laquelle lesdits cardiomyocytes sont sélectionnés dans le groupe constitué de cardiomyocytes de rat nouveau-né, de cardiomyocytes dérivés d'hiPSC, de cardiomyocytes dérivés d'hESC, de cardiomyocytes dérivés de cellules souches adultes, de cardiomyocytes dérivés de transdifférenciation, et de cardiomyocytes primaires humains.

5. Structure selon l'une quelconque des revendications 1 à 4, comprenant en outre des composants de matrice exogène ou des biomatériaux ajoutés sélectionnés dans le groupe constitué de polymères synthétiques, de polymères naturels, de collagène I, de collagène IV, d'acide hyaluronique, de gélatine et d'autres composants de matrice extracellulaire, ou des mélanges de ceux-ci.

6. Structure selon l'une quelconque des revendications 1 à 5 pour une utilisation dans le traitement d'une maladie cardiaque, de préférence sélectionnée dans le groupe constitué d'une maladie coronarienne et de la cardiomyopathie dilatée.

7. Procédé de production d'une structure cardiovasculaire *in vitro* selon la revendication 1, comprenant :
a) fournir des hASC, des cardiomyocytes, et, éventuellement des cellules endothéliales ;
b) cultiver lesdits hASC, éventuellement, avec lesdites cellules endothéliales ;
c) cultiver lesdits cardiomyocytes par dessus la culture formée dans l'étape b) ; et
d) administrer un VEGF et un FGF-2 à la culture cellulaire formée dans l'étape c).

8. Procédé selon la revendication 7, dans lequel les cellules endothéliales sont sélectionnées dans le groupe constitué de cellules endothéliales de veine ombilicale humaine, de cellules endothéliales micro-vasculaires humaines, de cellules endothéliales dérivées de cellules souches adipeuses humaines, de cellules endothéliales dérivées de cellules souches embryonnaires humaines, de cellules endothéliales dérivées de cellules souches pluripotentes induites, de cellules endothéliales dérivées de transdifférenciation, et de cellules progénitrices endothéliales.

9. Procédé de détermination d'une activité biologique d'une substance test, comprenant les étapes de :
a) fournir une structure cardiovasculaire *in vitro* selon la revendication 1 ;
b) administrer ladite substance test à ladite structure ou plateforme ;
c) déterminer l'effet de la substance test dans ladite structure ou plateforme ; et
d) comparer l'effet déterminé dans l'étape c) à un effet correspondant déterminé en l'absence de ladite substance test.

10. Procédé selon la revendication 9 dans lequel l'activité biologique à déterminer est sélectionnée dans le groupe constitué d'une toxicité cellulaire, d'une activité de modulation de formation de tubules, de propriétés électriques tel qu'un taux de régularité de contraction de cardiomyocyte, d'une durée de temps de repolarisation, de la présence d'arrhythmogénicité, de propriétés mécaniques telle que la force de contraction de cardiomyocyte et le métabolisme cellulaire.
